# NEW EUROPEAN PATENT SPECIFICATION

(11) **EP 0 355 099 B2**
(45) Date of publication and mention of the opposition decision: **04.12.1996**
(45) Mention of the grant of the patent: 28.07.1993
(21) Application number: 88902897.3
(22) Date of filing: 31.03.1988
(51) Int. Cl.: G01N 33/53, G01N 33/543, G01N 33/569, G01N 33/576

(54) **IMMUNOGLOBULIN ASSAY METHOD**
IMMUNOGLOBULIN-BESTIMMUNGSVERFAHREN
PROCEDE D'ANALYSE D'IMMUNOGLOBULINES

(30) Priority: 02.04.1987 GB 8707839
(43) Date of publication of application: 28.02.1990
(73) Proprietor: The Public Health Laboratory Service Board, London NW9 5EQ (GB)
(72) Inventor: MORTIMER, Philip, Paul, London NW7 4BP (GB); PARRY, John, Victor, Eastcote, Pinner Middlesex HA5 2JE (GB)
(74) Representative: Skelton, Stephen Richard
(86) International application number: GB8800251
(87) International publication number: WO8807680

(56) References cited:
- EP-A- 0 101 166
- EP-A- 0 132 170
- EP-A- 0 170 746
- EP-A- 0 211 756
- GB-A- 2 026 691
- Journal of Infectious Diseases, vol.155 no.4, April 1987, The University of Chicago. D.W.Archibald et al.:pages 793-796,
- Stevens. Archives of Diseases in Childhood.vol.61 pp 263-269. 1986.
- French et al. AIDS. vol 8(7). pp.885-894.1994
- Lange et al, Arztl.Lab.31.pp. 361-366.1985.
- J. Clin. Microbiology. vol. 24. pp. 288-293. 1986. Gerling et al.
- Packungsbeilage der Fa. Biomedicazum IgM Anti-Hbc-Enzyme Immunoassay Kit. 1986.
- Coyle & Sibony, Investigative Ophthalmology & Visual Science. vol 27, pp.622-625. 1986.
- Van Kan et al. J. Immunological Methods. vol. 57 pp.51-57.1983.
- Carlsson et al. J. Infectious Diseases, vol. 152. pp.1238- 1244. 1985.

## Description

This invention relates to methods of assay of immunoglobulins in bodily fluids. More specifically the invention provides a method of determining qualitatively or quantitatively the presence or amount of specific IgM, IgG or IgA in bodily fluids.

The diagnosis of many microbial infections, particularly those due to viruses, and the determination of immunity and susceptibility to them, is based on tests on serum. Serum is the fluid that remains when blood is allowed to clot and it is rich in proteins called immunoglobulins. These proteins are produced in response to infection by particular micro-organisms and are very specific to them. In this context immunoglobulins are referred to as antibodies. In the first few weeks after exposure to a microbial infection specific antibodies rise rapidly in concentration. The initial response to infection usually occurs in a particular class of immunoglobulin (IgM), but production of this sort of antibody is short lived so that it is usually present only when infection has been recent. It can therefore be used as a diagnostic sign, and is so used in a range of viral and other infections.

Another class of immunoglobulin (IgG) is formed a little later after infection. IgG antibodies are as specific as IgM antibodies but are much more persistent and, in the case of many viral infections, remain for life. IgG antibody is therefore a sign of infection with or immunity to the virus for which it is specific and its absence a sign of susceptibility. Among the infections for which measurement of specific IgM and IgG antibody are in common use are Rubella, Hepatitis A, Hepatitis B, Human Parvovirus B19, Measles, Mumps, Cytomegalovirus and Chicken Pox. It is becoming increasingly desirable to test for IgM and IgG antibodies for the virus responsible for the disease AIDS (eg HIV, HTLV III or LAV) and other retroviruses such as HTLV I.

It is also desirable in some cases to test for a further class of specific immunoglobulin, IgA, although this is generally less suitable for diagnostic or assay purposes. At present testing for the presence of specific IgG and IgM is carried out on blood serum, a bodily fluid which contains a very high concentration of IgG and IgM. A suitable serum test is for example described in J Medical Virology 19 (1986) p387-397. GB 2026691A describes an assay for blood serum IgG, IgM and IgA in which the Ig is captured by an anti-γ, µ or α immobilised on a substrate and then detected with a directly or indirectly labelled antigen. EPA 0132170 and 0101166 also describe assays for serum Ig using different methods.

Unfortunately serum cannot be collected without venepuncture and this is inconvenient and sometimes unacceptable, especially in children and nervous adults. In the case of tests for AIDS virus there is indeed widespread public prejudice against venepuncture. Venepuncture is also quite expensive, requiring a sterile needle, syringe, skin cleanser and dressing, and in certain cases it is hazardous and difficult.

Specific immunoglobulins are known to be present in other bodily fluids, some of which are much more accessible than serum, for example saliva, tears, semen, urine and cerebro spinal fluid. Concentration of IgM and IgG in these fluids are much lower than in serum eg in the order of 0.01 and 0.001 of the concentration in serum. Saliva is generally one of the most accessible of such fluids.

Some work has been carried out on characterising immunoglobulins in saliva, and on methods of assaying them. Much of this work has been of a general nature. For example Oral. Surg Oral Medicine Oral Pathol (US), (1985), 60(4), p372-376, J-Immunol. Meth. (1983), 57 (1-3), p51-7 and Invest. Opthamol Visual Sci (1986) 27 (4), P622-5 demonstrate the presence of all classes (IgA, IgG and IgM) in saliva.

J. Immunological Methods (1984) 71 203-10 demonstrate the presence of anti-Escherichia coli IgG and IgM antibodies in serum, by depositing specific Escherichia coli antigen on a substrate and exposing this to serum. This reference speculates that the technique could be used to test for these antibodies in saliva, but without any experimental evidence.

J-Infect. Diseases (US) (april 1987) 155 (4) p793-796 and (1985), 152 (6), p1238-44 describe attempts to assay IgA in saliva. The 1987 reference uses a technique that is suitable for research applications, but not for routine clinical work, which involves gel chromatography of the immunoprecipitate formed. The 1985 reference immobilises a specific poliovirus antigen on a substrate and attempts to capture specific IgA. Both of these references refer to the need to centrifuge the sample first.

The main immunoglobulins used in immunoglobulin assays, IgG and IgM, occur in saliva at only about 0.3% of the level of IgA in saliva, and thus none of the methods referred to above are expected to be useful for assay of IgG or IgM in saliva, or for that matter in other bodily fluids containing such low levels of IgG or IgM. In fact no presently used test or assays for specific IgG and IgM have been very successful or reliable when applied to either saliva or such fluids because of this low concentration, and hitherto no suitable method has been devised for assaying IgG or IgM in such fluids without inconvenient prior treatment such as centrifuging or concentration etc.

It is an object of the invention to provide a qualitative and quantitative test (assay) for specific immunoglobulins in readily accessible bodily fluids, particularly saliva.

According to the invention there is provided a method of assaying specific immunoglobulins of any or all classes present in a human or animal bodily fluid selected from saliva, semen, urine and cerebro spinal fluid, which comprises the steps of:
(i) immobilising an antibody to immunoglobulin G onto a solid substrate
(ii) exposing the immobilised antibody to a sample of the bodily fluid so as to achieve binding of a proportion of any immunoglobulin G in present in the fluid to the immobilised antibody, and either:
   (a) exposing the bound immunoglobulin G to a selected labelled antigen so as to achieve binding of the labelled antigen to at least a proportion of the bound immunoglobulin G or
   (b) exposing the bound immunoglobulin G to a selected antigen so as to achieve binding of the antigen to at least proportion of the bound immunoglobulin G and then binding a labelled antibody with specificity for the selected antigen directly or indirectly to at least a proportion of the bound antigen
(iii) and then detecting and/or measuring a property of the labelled antigen or antibody.

By "labelled" is meant an antigen or antibody having a detectable and/or measurable characteristic property. This property may be present in a known antigen or antibody or may be chemically or physically introduced into the antigen or antibody. The technique of labelling, and suitable labels are well known, and any conventional label may be used. Suitable labels include radio, enzyme, fluorescence or luminescence labelling, surface plasmon resonance (SPR), or attachment to small particles, such as of latex or gelatin, which have some detectable characteristic property.

By "selected antigen" is meant an antigen to which it is desired to demonstrate the presence of specific immunoglobulin in the bodily fluid.

By the method of the invention the detectable and/or measurable property of the labelled antigen or antibody may be qualitatively or quantitatively associated with the presence and/or amount of the specific immunoglobulin in the bodily fluid.

A significant factor of the method of the invention is that the possibility of assay depends not so much on the absolute concentration of the specific immunoglobulin in the bodily fluid but on the proportion of the total immunoglobulin that is specific to the bound antibody.

Using the method of the invention the presence and/or amount of a specific immunoglobulin in a bodily fluid in which it is present in relatively small amounts may be detected and/or measured. Of these saliva is preferred by virtue of convenience of availability.

By means of the invention many human and/or animal immunoglobulins may be assayed, for example those produced in response to the viruses which are associated with Hepatitis (A and B), Rubella, Measles, Human Parvovirus B19, Mumps, Chickenpox (VZ virus), and AIDS (HIV, LAV, or HTLV III).

Class-specific and non class-specific antibodies to immunoglobulins which may be used in step (i) of the invention are known, and may be commercially available or may be prepared using known methods, eg monoclonal methods. Suitable examples include the known anti IgG antibodies for example those which are specific for the immunoglobulin γ chains, found in IgG.

The antibody may be bound to the substrate using known methods, eg immersion of the substrate in a solution of the antibody at an appropriate dilution and pH. For example a pH of 7 to 11, especially 9 to 10 may be used, achieved with known buffers eg a Na₂CO₃/NaHCO₃ buffer. Solutions of antibodies are available commercially, and a suitable dilution is 1:200 to 1:4000 of the commercial antibody solution. Suitable substrates include polystyrene eg in bead form, or in the form of a microtitre plate or other small vessel.

The sample of bodily fluid for assay may be collected by entirely conventional techniques well known to those skilled in the art. In the case of the preferred fluid, saliva, it is preferred that the saliva is collected by allowing the patient to chew on a roll of sponge or cotton wool, which may subsequently be compressed or centrifuged to collect clarified saliva. There is hence generally no need to concentrate the immunoglobulins or further centrifuge the saliva prior to use of the assay method.

The substrate bearing the bound antibody may then be exposed to a sample of the bodily fluid, eg saliva, which may if necessary be diluted to an appropriate level with a buffer or may sometimes be used undiluted. Typically but not exclusively the fluid may be diluted 1:2 to 1:20 in the buffer. For commonly sought immunoglobulins. eg the IgG produced in response to the infections Rubella, Measles, Mumps, Hepatitis A or B, AIDS (ie HIV), dilution of the saliva sample in the range 1:2 to 1:10 in the buffer are generally suitable. A suitable buffer is phosphate buffered saline (PBS), containing 0.06 - 0.10 wt% sodium azide, 0.05 - 0.15% Tween 20 (trade mark) and 5 - 15% foetal calf serum (FCS). The conditions of exposure, eg time, temperature etc will depend upon the fluid, the immunoglobulin to be assayed, the bound antibody, the substrate etc, but can be made sufficiently short to enable the method to be used conveniently in clinical tests and screening. For example with the commonly sought immunoglobulins mentioned above, incubation with the diluted or undiluted fluid, eg saliva, sample for around 1 to 3 hours at 37°C is generally suitable. Excess bodily fluid solution is then washed away, eg with a solution of Tween 20 in PBS.

If alternative (a) of the method is to be used, the bound material may then be exposed to a solution of the labelled antigen, for example a radio- or enzyme- labelled antigen of one or more of the viruses referred to above. These may be prepared by known techniques (eg Schmitz et al J Gen Virol 50 1980)) and may be commercially available.

In alternative (a) of the method, it is generally preferable to use as pure a form of the antigen as can be obtained, to avoid the possibility, of material other than the antigen binding to the bound immunoglobulin, for example debris remaining from the medium in which the antigen was prepared.

A preferred form of labelled antigen for use in alternative (a) of the method is a small particle coated with the antigen. Many such particles are commercially available. If such particles have some detectable property such as radioactivity or in particular colour the binding of the particles to the bound immunoglobulin may be easily observed and/or measured.

If the particles are conspicuously coloured for example red or blue, then if the substrate consists of the surface of a well in a plate then the relative degree of binding of the particles may be measured by observing the intensity of the colour caused by the binding of these particles. Alternatively if the surface is shaped or aligned so that the force of gravity tends to prevent their binding to the surface, the extent to which they settle toward lower parts of the plate may be observed, for example with the naked eye or a microscope.

As the use of such coloured particles requires no sophisticated detection equipment such as radioactivity detectors, it is particularly convenient for use in the field or by relatively unskilled medical staff.

Alternative (b) is preferred inter alia because it does not necessarily require the use of as pure an antigen as is need for alternative (a). In this case the bound material is exposed to an unlabelled antigen eg an antigen of one or more of the viruses referred to above. Exposure conditions will depend upon the antigen used, etc but as above may be such as to allow convenient clinical use. Typically a commercially available solution of the antigen may be used, diluted with the buffer referred to above if necessary, which may also contain normal (ie which lacks the specific antibody sought in the assay) human serum, eg 0-5%.

After exposure to the unlabelled antigen, excess antigen solution should be washed away, eg with the buffer solution referred to above.

Binding of the labelled antibody to the bound antigen may be direct, eg by direct exposure of the bound antigen to the labelled antibody in solution. Alternatively the binding of the labelled antibody to the bound antigen may be indirect, eg a further antibody to the bound antigen, eg anti-Rubella monoclonal antibody may be bound to the bound antigen by exposure of the further antibody eg by exposure of the labelled antibody to the further antibody.

Binding of a radio-labelled antibody to the bound antigen or the further antibody may be achieved by exposure of the bound antigen or further antibody to a solution of the radio-labelled antibody.

The solution may be a commercially available radio-labelled antibody, diluted if necessary with a buffer, for example a buffer contain PBS plus 0.15 - 0.25% Tween 20, 5 - 15% FCS, 0 - 15% normal rabbit serum (NRS), 0 - 10% normal goat serum (NGS) and 0 - 50% normal human serum (NHS). Excess solution containing the unbound radio-label should then be washed away.

Suitable radio-labels include ¹²⁵I, ³²P, ³H and ³⁵S. These labels may be introduced into the antibody molecule using known techniques eg that of Salicinski et al, J.Endorinology 81 (1979).

If a radio-label is used, the radioactivity of the labelled material is then measured using conventional methods.

If an enzyme - labelled antibody to the bound antigen or the further antibody is to be used, this too may be achieved by exposure of the bound antigen or further antibody to a solution of the enzyme labelled antibody. Typically a commercially available antibody - enzyme conjugate may be used, diluted if necessary, for example in a buffer such as one containing PBS, 0 - 50% FCS, 0.5 - 15% NHS, 0 - 10% normal rabbit serum (NRS), 0 - 0.3% Tween 20.

Excess solution containing unbound enzyme - labelled antibody should then be washed away, eg with PBS plus Tween 20.

Suitable enzyme labels include peroxidase enzyme, eg horse-radish peroxidase (HRPO) and phosphatase enzymes. If not commercially available, antibody - enzyme conjugates may be prepared by known methods.

Some of the steps from (and including) exposure of the bound antibody to the sample containing the bodily fluid onward may be carried out simultaneously.

If an enzyme label has been used in alternative (a) or (b) the enzyme activity of the labelled material may then be measured, for example by allowing the bound enzyme label to catalyse a reaction the progress of which may be measured quantitatively and related to the amount of bound label present. Preferred reactions are those which may be followed photometrically, for example by the formation or disappearance of an optically absorbing compound. Such methods are conventional in the art.

Whether alternative (a) or (b) is followed and whatever form of label is used, the results eg radioactivity, enzyme activity from the assay or observation of colour eg from bound coloured particles may be treated in a conventional data processing manner. For example the results of an assay on a particular sample of fluid may be compared with standard negative samples known to contain none of the specific immunoglobulin, or may be compared with a set of positive samples known to contain a range of units of the specific immunoglobulin. Suitable processing methods will be well known to those skilled in the art.

To assist in using the invention in practice, kits may be provided containing for example samples of the necessary reagents, eg antibody for step (i), selected labelled antigen for step (ii)a, selected antigen for step (ii)b, labelled antibody for step (ii)b. The kit may also contain a chart for interpreting the results of step (iii), eg the appearence of the substrate if coloured particles are used in step (ii)a. The reagents etc may be supplied in such a kit at a suitable dilution as discussed above, and the kit may contain a substrate having antibody already immobilised thereupon for step (i).

For the avoidance of doubt, references to a l:n dilution herein (eg 1:1000 etc) mean 1 volume of liquid made up to n volumes with the diluent.

The invention will now be described by way of example only with reference to the following figures.

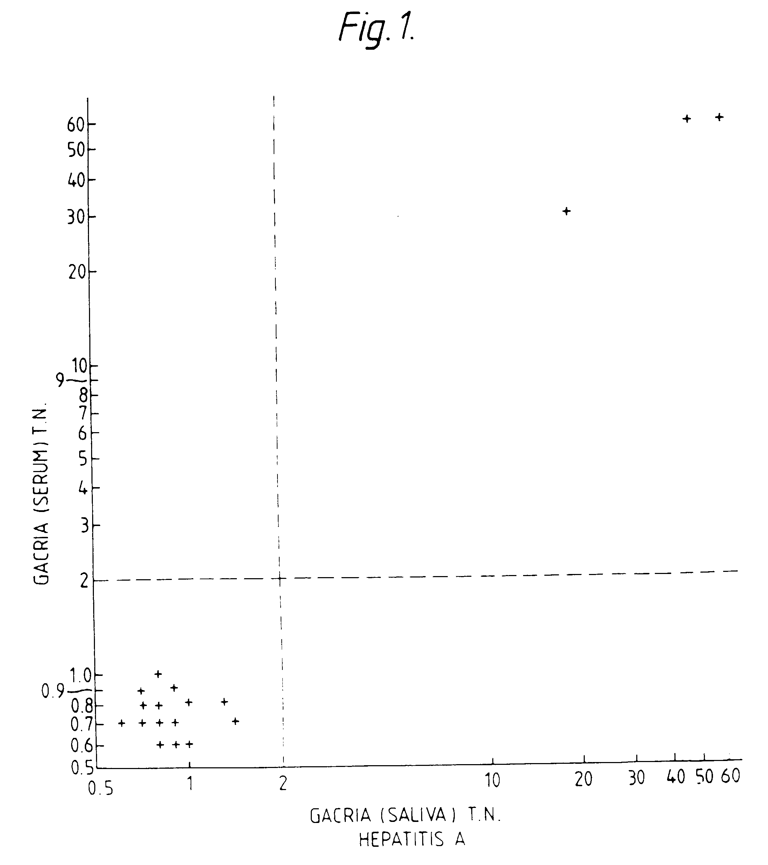

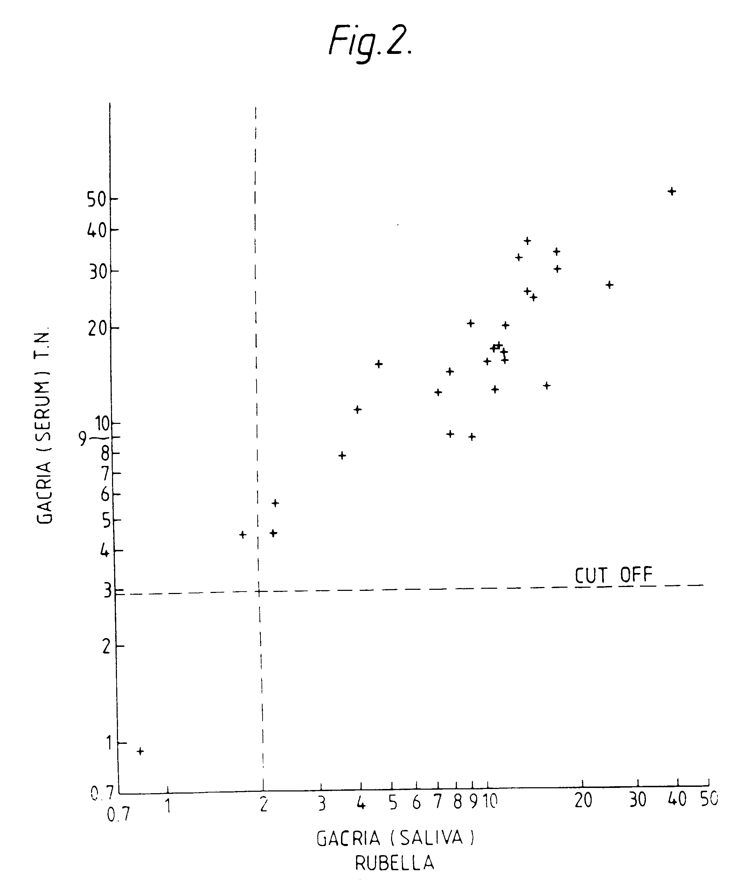

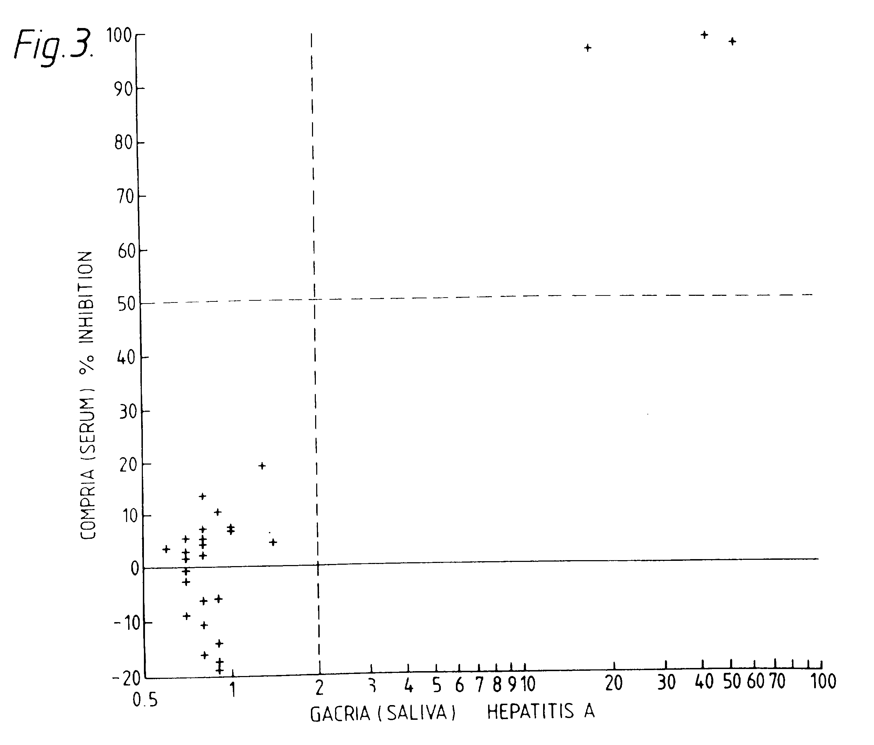

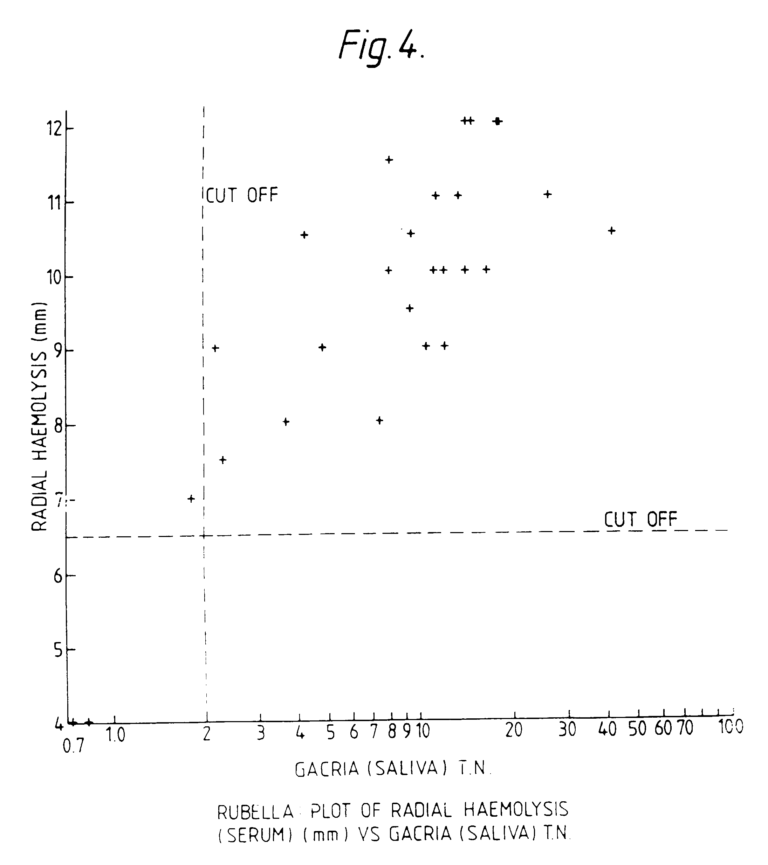

Figures 1 to 4 show comparisons between IgG assays using the method of the invention and assays using known methods on serum.

In the following examples, Assay Examples 1, 2, 3, 4 and 5 illustrate assays for IgG in saliva samples. Example 5 uses the method of alternative (a) and the remainder use that of alternative (b). Clinical examples are also described to illustrate the use of invention in medical practice.

The following terms used in this description are Trade Marks: Dako, Pentawash, Sterilin, Tween, Elavia.

### Assay Example 1

### Rubella IgG Capture radioimmunoassay (GACRIA) and enzymeimmunoassay (GACELISA) on saliva samples.

1. Coat polystyrene beads by immersion in 1/1000 Dako Anti-IgG (γ-chain), diluted in coating buffer (0.01 M sodium carbonate, 0.01 M sodium bicarbonate buffer, pH 9.6).
2. Prepare a 1/2 dilution of each saliva sample in phosphate buffered saline (PBS) with 0.08% sodium azide, 0.1% Tween 20, and 10% foetal calf serum (FCS).
3. Incubate the samples with the anti - IgG coated beads for 3 hours at 37°C.
4. Wash the beads four times in PBS with 0.1% Tween 20 (PBST), using the pentawash system (Abbott Laboratories). Add a 1/12 dilution of Rubella HA antigen (prepared by the Division of Microbiological Reagents & Quality Control, CPHL. Note that the concentration of the prepared HA antigen may vary between batches). Dilute the antigen in PBS, 0.1% Tween 20 and 10% FCS. Incubate overnight at room temperature.

### A. Radioimmunoassay

5. Wash the beads four times in PBST, and incubate for 2 hours at 37°C with anti-Rubella monoclonal antibody ascites (1/50,000) diluted in PBS 0.1% Tween 20 (T₂₀) 10% FCS, 5% normal rabbit serum (NRS), and 2% normal human serum (NHS).
6. Wash four times in PBST and add ¹²⁵l labelled anti mouse IgG (10⁵ counts per minute in 190 µl diluent) in PBS, 0.2% T₂₀, 10% FCS, 5% NRS, 5% normal goat serum (NGS), and 2% NHS. Incubate for 2 hours at 37°C.
7. Wash four times in PBST, transfer the beads to Abbott tubes, and count the bound radioactivity for 5 minutes in a γ- counter.

### B. Enzyme immunoassay

### 1 - 4 As above.

5. Wash the beads four times in PBST. Add HRPO conjugated monoclonal anti-rubella diluted 1 in 50 or 1 in 100 in PBS (without azide), 5% FCS, 1% NHS, 1%NRS, 0.1 % Tween 20. Incubate at room temperature for 3 hours.
6. Wash the beads four times in PBST and transfer to plastic tubes. Add 240 µl of substrate, ie ortho phenylene diamine (OPD) to the 1 in 100 dilution and tetramethylbenzidine (TMB) to the 1 in 50 dilution. Incubate at room temperature for 45 minutes. Stop the reaction with 100 µl of 4N H₂SO₄.
7. Transfer 150 µl of each sample and a blank. Read the optical density (O.D) at 492 nm (OPD) or 450 nm (TMB).

### Assay Example 2

### Hepatitis A IgG capture radioimmunoassay (GACRIA) and enzyme -immunoassay (GAC - ELISA) on saliva samples

1. Coat polystyrene beads by immersion in 1/1000 Anti - IgG (DAKO, γ chain), diluted in coating buffer. (0.0.1M sodium carbonate, 0.01m sodium bicarbonate buffer, ph 9.6).
2. Prepare a 1/10 dilution of each saliva sample in phosphate buffered saline (PBS) with 0.08% sodium azide, 0.1% Tween 20, and 10% foetal calf serum.
3. Incubate the samples with the anti - IgG coated beads for 3 hours at 37°C.

### A. Radioimmunoassay

4. Wash the beads four times in phosphate buffered saline with 0.05% Tween 20 (PBST) using the pentawash system (Abbott Laboratories). Add a 1/25 dilution of hepatitis A antigen (HAV Ag) in PBS, 0.1% Tween 20 (T₂₀), 10% foetal calf serum (FCS), and 1% normal human serum (NHS). Incubate overnight at room temperature.
5. Wash the beads four times in PBST, and incubate for 3 hours at 37 °C with ¹²⁵I - labelled human anti - HAV, 10⁵ (counts per minute in 190 ul diluent) in PBS, 0.2% T₂₀, 50% FCS , 10% NHS and 5% NRS.
6. Wash four times in PBST, transfer the beads to counting tubes, and count the bound radioactivity for 5 minutes in a γ-counter.

### B. Enzyme - Immunoassay

### 1 - 3 As above.

4. Wash the beads four times in PBST using the pentawash system (Abbott Laboratories). Add a 1/15 dilution of HAV.Ag in PBS, 0.1% T₂₀, 10% FCS, and 1% NHS. Incubate overnight at room temperature.
5. Wash the beads four times in PBST. Add 1/100 dilution of anti - HAV HRPO Conjugate (prepared by the Division of Microbiological Reagents and Quality Control, Public Health Laboratory Service), in PBS, 0.2% T₂₀ 50% FCS, 5% NRS and 10% NRS. Incubate for 1 hour at 37°C.
6. Wash the beads four times in PBST, then transfer to Abbott tubes, and add 240 µl of OPD Substrate to each bead. Incubate in the dark for 45 minutes at room temperature and stop the reaction with 100µl 4N Sulphuric acid.
7. Transfer 150 µl of each sample, and a blank, to a microtitre tray and read the optical density (O.D.) at 492 nm.

### Assay Example 3

### Hepatitis B core (HBc) IgG capture radioiumunoassay (GACRIA) and enzyme - immunoassay (GACELISA) on saliva samples.

1. Coat polystyrene beads by immersion in 1/1000 DAKO Anti-IgG (γ-chain), diluted in coating buffer (0.01 M sodium carbonate, 0.01 M sodium bicarbonate buffer, pH 9.6).
2. Prepare a 1/10 dilution of each saliva sample in phosphate buffered saline (PBS) with 0.08% sodium azide, 0.1% Tween 20, and 10% foetal calf serum (FCS).
3. Incubate the samples with the anti-IgG, coated beads for 2 hours at 45°C.
4. Wash the beads four times in PBS with 0.1% Tween 20 (PBST) using the pentawash system (Abbott Laboratories). Add a 1/200 dilution of HBc antigen, in PBS, 0.1% Tween 20 (T20), 10% FCS, and 0.5% normal human serum (NHS). Incubate for 60 minutes at 45°C.

### A Radioimmunoassay.

5. Wash the beads four times in PBST, and incubate for 2 hours at 45°C with ¹²⁵I - labelled human anti HBc (10⁵ counts per minute in 190 µl diluent) in PBS, 0.2% T₂₀, 10% normal rabbit serum (NRS), 10% FCS, and 40% NHS.
6. Wash four times in PBST, transfer beads to counting tubes, and count the bound radioactivity for 5 minutes in a γ-counter.

### B Enzyme - immunoassay

### 1 - 4 As above.

5. Wash the beads four times in PBST. Add 1/100 dilution of anti-HBc HRPO Conjugate (prepared by the Division of Microbiological Reagents and Quality Control, CPHL), diluted in PBS (without azide), 0.2% T₂₀, 10% FCS, 5% NRS and 10% NHS. Incubate for 2 hours at 37°C.
6. Wash the beads four times in PBST, then transfer to Abbott tubes, and add 240 µl of OPD Substrate to each bead. Incubate in the dark for 45 minutes at room temperature and stop the reaction with 100 µl 4N Sulphuric acid.
7. Transfer 150 µl of each sample, and a blank, to a microtitre tray, and read the optical density (O.D) at 492 nm.

### Assay Example 4

### Human immunodeficiency virus (HIV) IgG capture radioimmunoassay (GACRIA) and enzyme-immunoassay (GAC - ELISA) on salivary samples

1. Coat polystyrene beads by immersion in 1/1000 DAKO anti-IgG (γ - chain), diluted in coating buffer (0.01 M sodium carbonate, 0.01 M sodium bicarbonate buffer, pH 9.6).
2. Prepare a 1/10 dilution of each saliva sample in phosphate buffered saline (PBS) with 0.08% sodium azide, 0.1% Tween 20 (_{T}20) and 10% foetal calf serum (FCS).
3. Incubate the samples with the anti -IgG coated beads for 3 hours at 37°C.
4. Wash the beads four times in PBS with 0.05% T₂₀ (PBST) using the pentawash system (Abbott Laboratories), Add a 1/80 dilution of HIV antigen* (HIV Ag) in PBS, 0.1% T₂₀, 10% FCS, and 1% normal human serum (NHS). Incubate overnight at room temperature.

* The optimum antigen dilution may vary between batches.

### A. Radioimmunoassay

5. Wash the beads four times in PBST, and incubate for 2 1/2 hours at 37°C with ¹²⁵I - labelled human anti - HIV (10⁵ counts per minute in 190 µl diluent) in PBS, 0.2% T₂₀, 10% FCS, 20% NHS, and 10% normal rabbit serum (NRS).
6. Wash four times in PBST, transfer beads to counting tubes, and count the bound radio activity for 5 minutes in a γ-counter.

### B. Enzyme - immunoassay

### 1 - 4 As above

5. Wash the beads four times in PBST. Add a 1/4 dilution of Wellcome human anti - HIV peroxide conjugate in PBS with 10% NHS. Incubate for 2 1/2 hours at 37°C.
6. Wash four times in PBST, transfer to Abbott tubes and add 240 µl of OPD Substrate to each bead. Incubate in the dark for 30 minutes at room temperature and stop the reaction with 100µl 4N sulphuric acid.
7. Transfer 150 µl of each sample, and a blank, to a microtitre tray and read the optical density (O.D) at 492 mm.

### Assay Example 5

### Human Immunodeficiency Virus (HIV) IgG capture particle assay on serum and saliva samples

1. Coat 'U' or flat-bottom microtitre plate wells with a 1/1500 dilution of DAKO anti-IgG (γ-chain) in coating buffer (0.01 M sodium carbonate, 0.01 M sodium bicarbonate buffer, pH 9.6).
2. Prepare a dilution(s) of the serum sample (range 1/10 - 1/2000) or a 1/5 dilution of each saliva sample in the well in phosphate buffered saline (PBS) with 0.08% sodium azide, 0.01% Tween 20 (T₂₀) and 10% foetal calf serum (FCS).
3. Incubate the samples in the anti-IgG coated plates for 1 to 3 hours at 37°C.
4. Wash the wells four times in PBS with 0.05% T₂₀ (PBST) using a proprietary microtitre plate washing system.
5. Prepare a working suspension of antigen coated particles (eg HIV-coated gelatin particles as supplied by Fujirebio/ Serodia but further diluted 1 in 20 in PBS with 2% FCS). Add 50 µl of suspension to each well.
6. Cover the plate and allow particles to settle overnight at room temperature.
7. (U-bottom plates) Score particle binding on a scale from 4+ = complete binding to 0 = complete particle settling. Scores of 2 to 4 are regarded as provisional positive reactions to be confirmed by other methods.
8. (Flat-bottom plates) Plates are tilted at 45° and binding of particles is judged by the degree of settling of particles at the bottom edge of the well, scored as in 7, above.
9. If required, parallel tests using control, uncoated particles may be done similarly.

### Clinical Experiments.

Experiments were carried out using the methods of assay examples 1-4 above using 6mm diameter beads to compare capture and non-capture assays on saliva and serum.

### Clinical Experiment 1.

Specimens of serum and saliva were obtained simultaneously from 29 members of laboratory staff and tested in parallel for antibodies to the following antigens: rubella, hepatitis A and hepatitis B core. A small set of serum/saliva pairs from patients and consenting staff were tested for antibodies against HIV (AIDS virus) antigen.

IgG assays on the saliva specimens were carried out using the methods of examples 1-5 above. Comparative assays were carried out on serum using GACRIA (eg Parry, J.Med. Virol.19 (1986) 387-397), COMPRIA (COMpetitive Radio Immunoassay)(eg. Parry, Med. Lab. Sci. 38 (1981) 303-311, Mortimer et al, BMJ 290 1176-78, Cohen et al, J. Virol. Meth. (1981) 2 181-192) and radial haemolysis (Kurtz et al, J. Hygiene (1980) 84 213-222).

The results showed the following:
(i) Specific IgG could be detected in both the serum and saliva of individuals for a range of infections.
(ii) Results of immunoglobulin assays on serum and saliva showed complete qualitative and close quantitative correlation. Correlations between assays on saliva (application of the method of the invention), and the same GACRIA and COMPRIA assays plus non-capture (radial haemolysis) assay on serum were found for rubella, hepatitis A and B and HIV (AIDS virus).

Figs 1-4 show plots of T:N ratios, ie. the ratio of the radioactivity of a test sample (T) to that of a negative standard (N) known to contain none of the specific IgG, obtained using GACRIA and COMPRIA on saliva, against T:N ratios obtained on the corresponding conventional serum assays.

Fig 1 shows correlation between Hepatitis A assays using GACRIA on saliva in the method of example 2(A) and GACRIA as applied to serum.

Fig 2 shows correlation between rubella assays using GACRIA on saliva as in the method of example 1(A) and GACRIA as applied to serum.

Fig 3 shows correlation between hepatitis A assays using the method of example 2(A) and a corresponding COMPRIA assay on serum.

Fig 4 shows correlation between rubella virus assays using the method of example 1(A) and a radial haemolysis test on serum.

### Clinical Experiment 2

Fairs of serum/saliva samples were tested by conventional tests (COMPRIA, radial haemolysis, Enzyme Linked Immuno Sorbent Assay (ELISA (Elavia)) (eg Pasteur Diagnostics, Rubenz G, Northumbria Biologicals) and IgG capture assays (GACRIA, GACELISA)) for antibody to Hepatitis A virus (100 pairs examined),HIV (53 pairs), Hepatitis B virus core (62) and rubella virus (30). Conventional assays failed to detect antibodies to the four viruses in the saliva of many (93/119) subjects whose serum contained these antibodies. The method of the invention detected the antibodies in the saliva of all subjects who were seropositive by conventional tests except for two saliva specimens which were falsely negative for anti HBc and one falsely negative for anti-rubella virus. There was no significant difference between GACRIA anti HIV and anti HBc reactivities in serum and saliva, but GACRIA anti HAV and anti rubella reactivities were stronger in serum than in saliva. GACRIA and GACELISA results on the salivas for the four viral antibodies correlated closely and GACELISA results on saliva for anti-HAV and anti HIV were as accurate as GACRIA results. Both for saliva and serum GACRIA was superior to IgA capture assays in detecting anti HAV and HIV.

### Materials and methods

(i) Materials For anti HAV (Hepatitis A virus) and anti rubella virus tests pairs of serum/saliva specimens were collected from travellers requesting prophylaxis against HAV and from laboratory staff. Each subject gave blood by venepuncture and salivated into a 3.5 cm diameter pot with a screw lid (sterilin). Paired blood and saliva specimens were similarly collected for anti HIV testing from high and low risk subjects. These specimens and those from laboratory staff were also tested for anti HBc (hepatitis B core). For testing the serum was separated from each clotted blood, saliva specimens were tested untreated. Serum and saliva were stored at -30°C.
(ii) Conventional tests Anti HAV, anti HIV and anti HBc were measured by COMPRIA. Results were expressed as percentage inhibition of binding of ¹²⁵I label. Cut off values were 50% inhibition for the anti HAV and the anti HIV, and 70% for the anti HBc. Anti rubella was measured by radial haemolysis. Anti HIV and anti rubella were also measured by ELISA and Elavia.
(iii) Capture assays 6 mm polystyrene beads were coated by immersion in a 1 : 1000 dilution of rabbit antiserum to human γ - or α-chain (DAKO immunoglobulins). Before use and between each step in the assays the beads were washed in PBS with 0.05 % Tween 20. Serum or saliva samples diluted with PBS with 0.05 % Tween 20 were incubated with the beads. Then HIV antigen HAV antigen, HBc antigen or rubella virus haemagglutinin was applied followed by a detector reagent. This reagent was purified human IgG with a high titre of anti HAV, anti HIV or anti HBc, or purified mouse monoclonal antibody to rubella, linked either to ¹²⁵I or to peroxidase. Non specific sticking of the detector reagent was diminished by adding human serum (specific antibody negative). Binding of the detector reagent was measured either as γ emissions or as the optical density of colour generated by the action of the enzyme on orthophenylene diamine or (for rubella) teramethylbenzidine. Reactions exceeding twice the negative control (T:N>2) were considered positive.
(iv) Results For each viral antibody results by capture assay for IgG antibody on serum and saliva were compared with results by conventional tests (table 1). Conventional test results on sera were as follows: for anti HAV 66 subjects negative, 34 positive (COMPRIA 84%- 98% inhibition, median 92 %), for anti HIV 10 subjects negative, 43 positive (COMPRIA 84% - 98%, Elavia all > 2.00), for anti HBc 37 subjects negative, 25 positive (COMPRIA 76% - 95%, median 91 %) and for anti rubella 3 negative, 27 positive (radial haemolysis 7 - 12 mm, median 10mm, Rubenz G 0.75 - 2.00, median 2.00). Results by conventional tests on the salivas of seropositive subjects were mostly negative. Rubella virus radial haemolysis and ELISA never detected antibody in saliva, and COMPRIA for anti HAV and anti HBc did so only in a few instances (3/32, 2/18).

With very few exceptions IgG antibody capture radioimmunoassay (GACRIA) results on serum and saliva specimens were qualitatively the same and matched conventional results on the sera. The exceptions were two subjects who had weak anti HBc and 1 who had weak anti-rubella reactivity in serum.These three were antibody negative in GACRIA tests on saliva.

Correlations between results obtained with conventional tests on serum and with GACRIA on serum and saliva were, except for anti-rubella not close (table 2). Closer correlations were observed between serum and saliva results by GACRIA except for anti HIV. For this antibody, the two sorts of specimens were not tested simultaneously as they were for the other three shown in table 2.

Using the enzyme immunoassay (GACELISA) results were qualitatively the same as for GACRIA and by conventional tests on the corresponding sera except for 4 false negative anti HBc and ten false negative anti rubella virus results . The 14 specimens in question were in almost all cases more reactive in GACELISA than the true negative specimens were, but they were less than twice the mean negative control value. GACELISA results on saliva correlated closely with GACRIA results except for anti HAV in which 21/32 GACELISA optical density values were > 2.00, the upper limit on the ELISA reader.

In view of the high concentration of IgA relative to IgG in saliva specific IgA and IgG capture assay tests of saliva were compared as a means of demonstrating anti HAV and anti HIV positivity. Specific IgA could only be found in the saliva of half of those tested though it was detected in the serum of all but two sero-positive individuals (table 3).

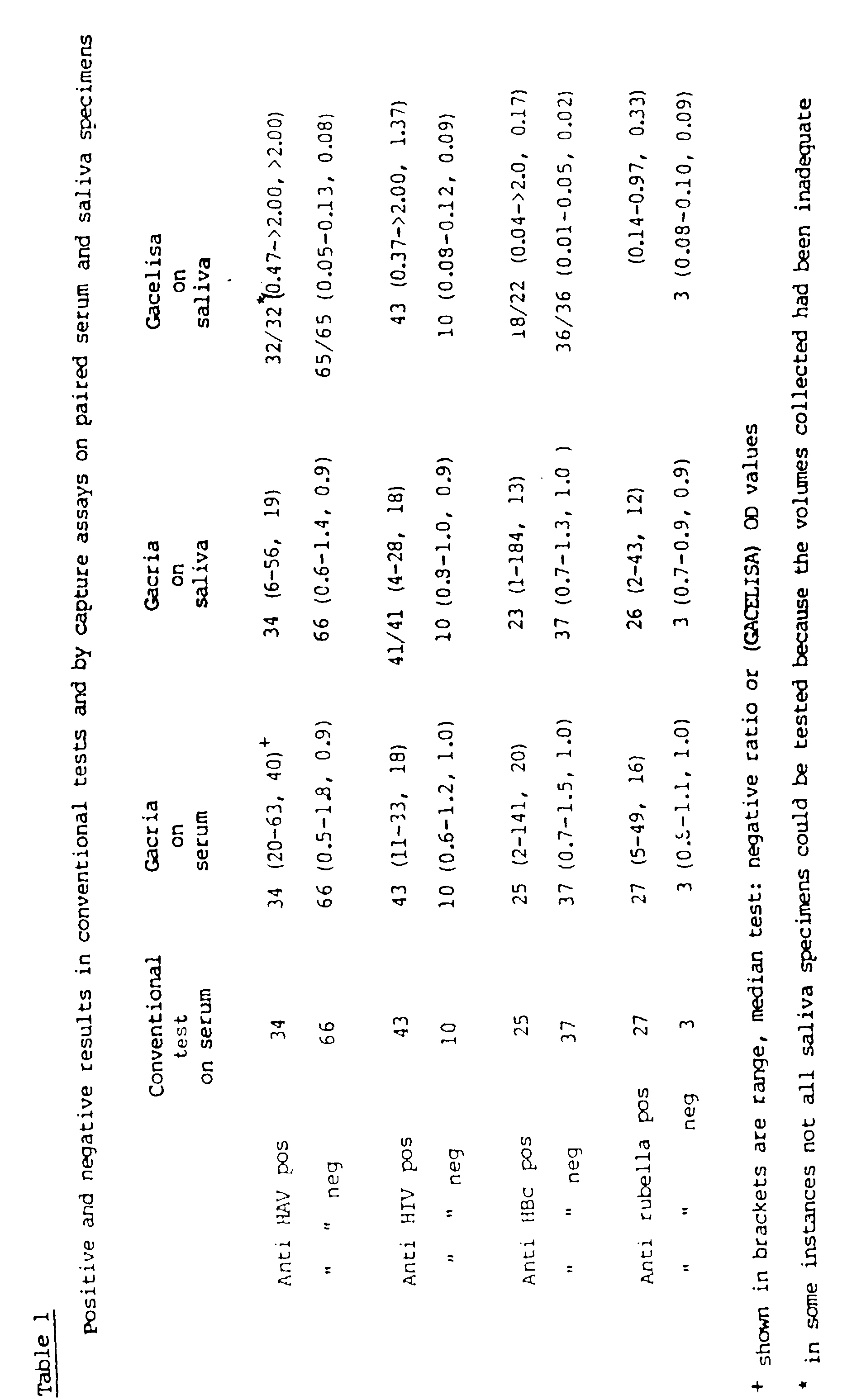

**Table 2**

| Correlation of GACRIA results on saliva with other results on serum and saliva specimens from seropositive individuals (correlation coefficient r is shown) | | | |
|---|---|---|---|
| Gacria on saliva versus: | Conventional test on serum⁺ | Gacria on on serum | Gacria Gacelisa on saliva |
| anti HAV | 0.36* | 0.71*** | 0.42* |
| anti HIV | 0.08 | 0.15 | 0.83*** |
| anti HBc | 0.27 | 0.95*** | 0.96*** |
| anti rubella | 0.49** | 0.84*** | 0.74*** |

| | | | |
|---|---|---|---|
| ⁺ COMPRIA results, for anti rubella RH. | | | |
| * Significant correlation p <0.05 : | | | |
| ** p <0.01 : | | | |
| *** p <0.001 | | | |

**Table 3**

| Comparison of Specific IgG (GACRIA) and specific IgA assay results on serum and saliva specimens from 10 anti HAV positive and 19 anti HIV positive subjects. | | | |
|---|---|---|---|
| | | Serum | Saliva |
| | IgG | 10 (74 - 155, 146)⁺ | 10 (50 - 113, 81) |
| anti HAV | | | |
| | IgA | 10 ( 7 - 50, 22) | 9 (1.9 - 35, 5.0) |
| | | | |
| | IgG | 19 (11 - 33, 16) | 19 (5 - 26, 20) |
| anti HIV | | | |
| | IgA | 17 (1.8 - 31, 4.4) | 5 (0.9 - 16, 1.4) |

| | | | |
|---|---|---|---|
| ⁺ number positive (range, median test: negative ratio) | | | |

### Clinical experiment 3

The method described in assay example 5 was used to detect anti HIV IgG in serum and saliva specimens.

170 coded serum specimens from individulas at increased risk of HIV infection were tested. 26 had been confirmed as anti HIV positive by conventional assays and all of these gave 4+ (complete) agglutination in the particle assay. Of the 144 known to be anti HIV negative, four gave trace (1+) agglutination and two 3+ to 4+ agglutination. On repeat testing all were negative except one serum specimen which still gave 4+ agglutination.

73 saliva specimens, 62 from patients known to have anti HIV in their serum, were tested under code. The salivas of 61 of the 62 seropositive patients were anti HIV IgG positive (56 gave 4+, 5 gave 3+ agglutination). Of the 11 seronegative patients' saliva two gave 4+ agglutination which was further investigated using uncoated (HIV negative) particles.

## Claims

1. A method of assaying a specific immunoglobulin G present in a human or animal bodily fluid selected from saliva, semen, urine and cerebro spinal fluid, which comprises
(i) immobilising an antibody to immunoglobulin G onto a solid substrate,
(ii) exposing the immobilised antibody to a sample of the bodily fluid so as to achieve binding of a proportion of any immunoglobulin G present in the fluid to the immobilised antibody, and either
a. exposing the bound immunoglobulin G to a selected labelled antigen so as to achieve binding of the labelled antigen to at least a proportion of the bound immunoglobulin G, or
b. exposing the bound immunoglobulin G to a selected antigen so as to achieve binding of the antigen to at least a proportion of the bound immunoglobulin G and then binding a labelled antibody with specificity for the selected antigen directly or indirectly to at least a proportion of the bound antigen, and
(iii) detecting and/or measuring a property of the labelled antigen or antibody while the labelled antigen or antibody is bound to the solid substrate.

2. A method according to claim 1, characterised in that the fluid is saliva.

3. A method according to claim 2, characterised in that the saliva is obtained from a patient and then used either undiluted or diluted but without a step of increasing the immunoglobulin concentration.

4. A method according to claim 1 or claim 2 characterised in that the alternative (ii)b is used.

5. A method according to claim 1 wherein the immunoglobulin to be assayed is produced in response to infection by Hepatitis A Hepatitis B, Human Immunodeficiency Virus (ie AIDS virus), Rubella, Measles or Mumps.

6. A method according to claim 1 wherein the immunoglobulin to be assayed is produced in response to Human Parvovirus B19 or Chickenpox (VZ virus).

7. A method according to claim 1 wherein a further antibody is bound to the bound antigen and the labelled antibody is bound to the further antibody.

8. A method according to claim 7 wherein the immunoglobulin to be assayed is produced in respnse to Rubella infection.

9. A method according to claim 1 wherein the labelled antigen is a gelatin or latex particle coated with the antigen.

10. A method according to claim 1 wherein the particle is conspicuously coloured.

11. A method according to claim 9 wherein the immunoglobulin to be assayed is one produced in response to infection by Human Immunodeficiency Virus.

12. Use of a kit for assaying specific immunoglobulin G present in a human or animal bodily fluid selected from saliva, semen, urine and cerebro spinal fluid using the method claimed of claim 1 wherein the kit comprises (i) a solid substrate having antibody to immunoglobulin G immobilised onto it and (ii) labelled antigen capable of binding to the specific immunoglobulin G.

13. Use of a kit according to claim 12 wherein the antigen is coated onto gelatin or latex particles.

14. Use of a kit for assaying specific immunoglobulin G present in a human or animal bodily fluid selected from saliva, semen, urine and cerebro spinal fluid using the method claimed of claim 1 wherein the kit comprises (i) a solid substrate having antibody to immunoglobulin G immobilised onto it, (ii) an antigen capable of binding to the specific immunoglobulin G and (iii) a labelled antibody with specificity for the said antigen.

## Patentansprüche

1. Verfahren zur Bestimmung eines spezifischen Immunglobulins G, das in einer unter Speichel, Sperma, Urin und Rückenmarksflüssigkeit ausgewählten menschlichen oder tierischen Körperflüssigkeit vorhanden ist, bei dem:
(i) ein Antikörper gegen Immunglobulin G an einem festen Substrat immobilisiert wird,
(ii) der immobilisierte Antikörper einer Probe der Körperflüssigkeit ausgesetzt wird, um die Bindung eines Teils eines beliebigen in der Flüssigkeit vorhandenen Immunglobulins G an den immobilisierten Antikörper zu erreichen, wobei entweder
a. das gebundene Immunglobulin G einem ausgewählten markierten Antigen ausgesetzt wird, um die Bindung des markierten Antigens an mindestens einen Teil des gebundenen Immunglobulins G zu erreichen, oder
b. das gebundene Immunglobulin G einem ausgewählten Antigen ausgesetzt wird, um die Bindung des Antigens an mindestens einen Teil des gebundenen Immunglobulins G zu erreichen, und anschließend ein markierter Antikörper mit Spezifität für das ausgewählte Antigen direkt oder indirekt an mindestens einen Teil des gebundenen Antigens gebunden wird, und
(iii) eine Eigenschaft des markierten Antigens oder Antikörpers nachgewiesen und/oder bestimmt wird, während das/der markierte Antigen oder Antikörper an das feste Substrat gebunden ist.

2. Verfahren nach Anspruch 1, dadurch gekennzeichnet, daß die Flüssigkeit Speichel ist.

3. Verfahren nach Anspruch 2, dadurch gekennzeichnet, daß der Speichel von einem Patienten erhalten wird und anschließend entweder unverdünnt oder verdünnt verwendet wird, jedoch ohne einen Schritt zur Erhöhung der Immunglobulin-Konzentration.

4. Verfahren nach Anspruch 1 oder 2, dadurch gekennzeichnet, daß die Alternative (ii)b angewendet wird.

5. Verfahren nach Anspruch 1, wobei das zu bestimmende Immunglobulin als Reaktion auf die Infektion mit Hepatitis A, Hepatitis B, humanem Immunschwäche-Virus (d.h. AIDS-Virus), Röteln, Masern, oder Mumps gebildet wird.

6. Verfahren nach Anspruch 1, wobei das zu bestimmende Immunglobulin als Reaktion auf den humanen Parvovirus B19 oder Windpocken (VZ-Virus) gebildet wird.

7. Verfahren nach Anspruch 1, wobei ein weiterer Antikörper an das gebundene Antigen gebunden wird, und der markierte Antikörper an den weiteren Antikörper gebunden wird.

8. Verfahren nach Anspruch 7, wobei das zu bestimmende Immunglobulin als Reaktion auf eine Röteln-Infektion gebildet wird.

9. Verfahren nach Anspruch 1, wobei das markierte Antigen ein mit dem Antigen beschichtetes Gelatine- oder Latex-Teilchen ist.

10. Verfahren nach Anspruch 1, wobei das Teilchen deutlich gefärbt ist.

11. Verfahren nach Anspruch 9, wobei das zu bestimmende Immunglobulin eines ist, das als Reaktion auf eine Infektion mit humanem Immunschwäche-Virus gebildet wird.

12. Verwendung eines Kits zur Bestimmung eines spezifischen Immunglobulins G, das in einer unter Speichel, Sperma, Urin und Rückenmarksflüssigkeit ausgewählten menschlichen oder tierischen Körperflüssigkeit vorhanden ist, unter Anwendung des Verfahrens nach Anspruch 1, wobei der Kit aufweist:
(i) ein festes Substrat mit einem darauf immobilisierten Antikörper gegen Immunglobulin G und
(ii) ein markiertes Antigen, das an das spezifische Immunglobulin G binden kann.

13. Verwendung eines Kits nach Anspruch 12, wobei das Antigen auf Gelatine- oder Latex-Teilchen aufgebracht ist.

14. Verwendung eines Kits zur Bestimmung eines spezifischen Immunglobulins G, das in einer unter Speichel, Sperma, Urin und Rückenmarksflüssigkeit ausgewählten menschlichen oder tierischen Körperflüssigkeit vorhanden ist, unter Anwendung des Verfahrens nach Anspruch 1, wobei der Kit aufweist:
(i) ein festes Substrat mit einem daran immobilisierten Antikörper gegen Immunglobulin G,
(ii) ein Antigen, das an das spezifische Immunglobulin G binden kann, und
(iii) einen markierten Antikörper mit Spezifität für das Antigen.

## Revendications

1. Procédé d'analyse et de dosage d'une immunoglobuline G spécifique présente dans un fluide corporel humain ou animal, fluide choisi parmi de la salive, de la semence, de l'urine et du fluide cérébro-spinal, ce procédé comprenant :
(i) l'immobilisation d'un anticorps de l'immunoglobuline G sur un substrat solide,
(ii) l'exposition de l'anticorps immobilisé à un échantillon du fluide corporel de façon à réaliser la liaison, sur l'anticorps immobilisé, d'une certaine proportion de l'immunoglobuline G éventuellement présente dans le fluide, et
(a) ou bien exposition de l'immunoglobuline G, ainsi fixée, à un antigène choisi et marqué, de façon à réaliser la liaison de l'antigène marqué sur au moins une certaine proportion de l'immunoglobuline G liée, ou
(b) l'exposition de l'immunoglobuline G, liée ou fixée, à un antigène choisi, de façon à réaliser la liaison de l'antigène sur au moins une certaine proportion de l'immunoglobuline G fixée, puis la liaison d'un anticorps marqué ayant de la spécificité pour l'antigène choisi, directement ou indirectement sur au moins une certaine proportion de l'antigène fixé, et,
(iii) déceler et/ou mesurer une propriété de l'antigène ou de l'anticorps marqué, pendant que l'antigène ou l'anticorps marqué est fixé sur le substrat solide.

2. Procédé selon la revendication 1, caractérisé en ce que le fluide est de la salive.

3. Procédé selon la revendication 2, caractérisé en ce que la salive est obtenue sur un patient et elle est ensuite utilisée non diluée ou bien diluée mais sans une étape d'augmentation de la concentration de l'immunoglobuline.

4. Procédé selon la revendication 1 ou la revendication 2, caractérisé en ce que l'on utilise le terme (ii)b de l'alternative.

5. Procédé selon la revendication 1, dans lequel l'immunoglobuline à analyser et/ou à doser est produite en réponse à une infection par de l'hépatite A, de l'hépatite B, le virus de l'immunodéficience humaine (c'est-à-dire le virus du SIDA), la rubéole, la rougeole ou les oreillons.

6. Procédé selon la revendication 1, dans lequel l'immunoglobuline à analyser et/ou à doser est produite en réponse à l'action du Parvirovirus humain B19 ou à la varicelle (virus VZ).

7. Procédé selon la revendication 1, dans lequel un anticorps supplémentaire est fixé sur l'antigène fixé, et l'anticorps marqué est fixé sur l'autre anticorps.

8. Procédé selon la revendication 7, dans lequel l'immunoglobuline à analyser et/ou à doser est produite en réponse à une infection de rubéole.

9. Procédé selon la revendication 1, dans lequel l'antigène marqué est une particule de gélatine ou de latex revêtue de l'antigène.

10. Procédé selon la revendication 1, dans lequel la particule est remarquablement colorée.

11. Procédé selon la revendication 9, dans lequel l'immunoglobuline à analyser et/ou à doser est une immunoglobuline produite en réponse à une infection par le virus de l'immunodéficience humaine (virus du SIDA).

12. Utilisation d'un nécessaire pour analyser et/ou doser de l'immunoglobuline G spécifique présente dans un fluide corporel humain ou animal, fluide choisi parmi de la salive, de la semence, de l'urine et du fluide cérébrospinal, en utilisant le procédé revendiqué à la revendication 1, utilisation dans laquelle le nécessaire comprend (i) un substrat solide sur lequel est immobilisé un anticorps de l'immunoglobuline G, et (ii) l'antigène marqué capable de se fixer sur l'immunoglobuline G spécifique.

13. Utilisation d'un nécessaire selon la revendication 12, dans laquelle l'antigène est appliqué en revêtement sur des particules de gélatine ou de latex.

14. Utilisation d'un nécessaire pour analyser et/ou doser de l'immunoglobuline G spécifique présente dans un fluide corporel humain ou animal, choisi parmi de la salive, de la semence, de l'urine et du fluide cérébro-spinal en utilisant le procédé revendiqué à la revendication 1, utilisation dans laquelle le nécessaire comprend (i) un substrat solide sur lequel est immobilisé de l'anticorps à l'égard de l'immunoglobuline G, (ii) un antigène capable de se fixer sur l'immunoglobuline G spécifique et (iii) un anticorps marqué ayant de la spécificité pour ledit antigène.
